# EUROPÄISCHE PATENTSCHRIFT

(11) **EP 0 371 424 B1**
(45) Veröffentlichungstag und Bekanntmachung des Hinweises auf die Patenterteilung: **01.06.1994**
(21) Anmeldenummer: 89121832.3
(22) Anmeldetag: 25.11.1989
(51) Int. Cl.: A61B 5/08, A61B 5/0404, A61B 5/0205, A61F 5/56

(54) **Überwachungsvorrichtung zur Diagnose von Apnoe**
Monitoring device for the diagnosis of apnea
Dispositif de surveillance destiné au diagnostic de l'apnée

(30) Priorität: 30.11.1988 US 278139
(43) Veröffentlichungstag der Anmeldung: 06.06.1990
(73) Patentinhaber: MADAUS SCHWARZER MEDIZINTECHNIK GmbH & Co. KG, D-81245 München (DE)
(72) Erfinder: Griebel, Peter, D-7800 Freiburg (DE)

(56) Entgegenhaltungen:
- WO-A-88/10108
- DE-A- 2 839 331
- FR-A- 2 377 793
- US-A- 4 220 142
- MEDICAL AND BIOLOGICAL ENGINEERING AND COMPUTING. vol. 24, no. 02, März 1986, STEVENAGE GB Seiten 182 - 185; J.P. HEEMELS et al.: "Tracheal sound detector"
- IEEE Computers in Cardiology, September 12-15, 1987, LEUVEN/Belgium. The Computer Society of the IEEE Seiten 411 - 414; Y. ICHIMARU et al.: "Detection of periodic breathing during 24-hour ambulatory ECG monitoring"

## Beschreibung

Die Erfindung betrifft ein ambulantes Diagnosesystem zur unverzüglichen Behandlung von Schlafapnoe nach dem Oberbegriff des Anspruchs 1.

Das Zusammentreffen eines verminderten aktiven Wechsels der Herzfrequenz und des Atmungspotentials über eine beträchlich lange Zeit und einer darauffolgenden erhöhten Herzfrequenz gilt als Indikation für Schlafapnoe. Wie schon in den US PS 4, 422,458 und 4,580,575 bemerkt wird, ist diese Aufzeichnungsart jedoch von elektrischen Spannungsgeräten abhängig, die die Nützlichkeit dieser Signale beeinträchtigen. Je nach der Form des Auftretens kann auch Schnarchen selbst ein Risikofaktor beim Verdacht auf Obstruktionsschlafapnoe sein und die erwähnten Geräte können dies nicht mitberücksichtigen. Verschiedene Arten der Schlafapnoe erfordern auch unterschiedliche therapeutische Maßnahmen. Die erwähnten Geräte bieten nicht die differenzierten Diagnose-möglichkeiten, die zur Behandlung eines Patienten auf Apnoe erforderlich sind.

Durch die US PS 4,715,367 ist es bekannt, die Atmung akustisch mit Hilfe eines Gerätes zur Feststellung von Veränderungen des Atmungsverhaltens zu überwachen. Das Gerät stellt lautes schnarchen fest oder weckt den Patienten während einer Phase langer Stille auf, die auf eine gefährlich lange, akute Episode von Schlafapnoe hindeutet. Weitere akustische Alarmgeräte sind in der US PS 4,306,567 und der US PS 4,129,125 offenbart.

Die DE-A-28 39 331 beschreibt ein tragbares Überwachungsgerät zur elektrokardiographischen Überwachung der Herzströme, das Mittel zur Abtastung der Herzspannung und Mittel zur Bestimmung der Herzfrequenz aus der Herzspannung sowie Mittel zur Aufzeichnung kodierter Signale, welche die Herzfrequenz darstellen, umfaßt.

Die FR-A-2 377 793 beschreibt eine Vorrichtung zur kontinuierlichen Überwachung der Atmung einer Person mit Mitteln zur Bestimmung der von der Atmung hervorgerufenen akustischen Schwingungen, die eine zu den Schwingungen proportionale elektrische Spannung erzeugen, Mitteln zur Verstärkung dieser elektrischen Spannung, Mitteln zur Übertragung der verstärkten elektrischen Spannung und Mitteln zur Auswertung der übertragenen Spannung.

Schnarchen gilt im allgmeinen als Indikation von Obstruktionsapnoe, die von neurologischer, organischer Apnoe zu unterscheiden ist. Das Auftreten von Schnarchen und Stille, entweder zusammen oder getrennt, bietet jedoch keine ausreichende Information, um eine Diagnose zur gezielten Behandlung von Schlafapnoe aufzustellen. Einige kurze, nicht-akute Schlafapnoeepisoden stören den Schlaf und rufen wachsende Ermüdungserscheinungen hervor, die klinisch bedeutend werden können. Es können jedoch auch ähnliche Stilleperioden festgestellt werden, die aufgrund des Unterschiedes zwischen Brust- und Bauchatmung jedoch keine Indikation für Schlafapnoe sind.

Eine unverzügliche und exakte Diagnose von Schlafapnoe ist ebenfalls von Bedeutung, da Schlafapnoe ein Zustand ist, der behandelt und sogar abgestellt werden kann, wenn er rechtzeitig festgestellt und die Ursache des Problems fühzeitig erkannt wird. Verschiedene Episoden von Schlafapnoe sind als solche nicht lebensbedrohlich, sie sind jedoch ein Risikofaktor für Herzpatienten. Frühe, exakte Diagnose und sofortige Behandlung von Schlafapnoe ist bei solch risiko-gefährdeten Patienten jedoch von besonderer Bedeutung.

Bei Verdacht auf Schlafapnoe wird der Patient daher meist in das Schlaflabor einer Klinik überwiesen, wo Möglichkeiten vorhanden sind, den Zustand des Patienten während seines Schlafes mit Hilfe einer polysomnographischen Aufnahme zu diagnostizieren. Polysomnographische Auswertungen müssen allerdings stationär vorgenommen werden - sie beinhalten eine Überwachung des Luftdruckes in der Brust, des Blut-Sauerstoff Teildruckes (P) und des gesättigten Blut-Sauerstoffdruckes, des Bauch- und Brustatmungspotentials, der Herzfrequenz und auch anderer möglicher Indikatoren.

Ein solcher Krankenhausaufenthalt ist natürlich kostenaufwendig und und stört den Lebensrhythmus des Patienten, was an sich schon zu Schlafstörungen führen kann. Solche zusätzlichen Störungen können unter Umständen zu irrigen Ergebnissen führen und möglicherweise die Diagnose der eigentlichen Ursache der klinischen Symptome des Patienten beeinträchtigen. Die nichtautomatisierte Auswertung der umfangreichen, detaillierten Aufzeichnungen ist überdies hoch technisch und sehr zeitaufwendig.

Es wurde überraschenderweise festgestellt, daß der erfindungsgemäße automatisierte Bericht trotz seiner größeren Einfachheit und Kompaktheit keine falschen negativen Indikationen hervorbringt wenn er von medizinisch geschultem Personal ausgewertet wird. Überraschenderweise wurde mit der Erfindung auch eine vollständige Diagnose für 54% der Testpatienten erzielt. Für den restlichen Teil war es dem medizinischen Personal aufgrund der Erfindung möglich, die Anzahl der Patienten für eine vollständige polysomnograohische Auswertung auf Fälle zu beschränken, bei denen eine organische Apnoe angezeigt ist.

Die Erfindung betrifft ein ambulantes Diagnosesystem zur unverzüglichen Behandlung von Schlafapnoe mit Mitteln zur Abtastung der Herzspannung, Mitteln zur Bestimmung der Herzfrequenz aus der Herzspannung und Mitteln zur Aufzeichnung kodierter Signale, welche die Herzfrequenz darstellen; es umfaßt weiterhin:
Mittel zur Erfassung von Atmungslauten, Mittel zur Erfassung von Schnarchlauten, Mittel zur Aufzeichnung kodierter Signale, welche die Atmungs- und Schnarchlaute darstellen und Mittel zur Berechnung des Atmungsregulationsstörungsindexes aus der Anzahl der Abschnitte, in denen die Herzfrequenz im wesentlichen durchschnittlich war und der Anzahl der Abschnitte, in denen Atmungslaute nicht erfaßt wurden, sowie
Mittel zur Formatierung der aufgezeichneten Signale zur Erstellung von Graphiken über die zeitliche Beziehung zwischen den Veränderungen, die in der aufgezeichneten Herzfrequenz des Patienten von Schnarch- und Atmungslauten des Patienten auftreten, und
Mittel zur Formatierung statistischer Graphiken aus den aufgezeichneten Signalen, wobei diese Graphiken die Herzfrequenzverteilung ebenso wiedergeben wie die Häufigkeitsverteilung der Dauer der Zeitabschnitte zwischen dem aufgezeichneten Auftreten von Schnarchlauten und der Dauer der Zeitahschnitte, in denen die Herzfrequenz konstant bleibt.

Gemäß der vorliegenden Erfindung werden aufgrund der codierten Signale der Herzfrequenz des Patienten zwei Atmungsstörungsindizes und das Auftreten von Atmungslauten des Patienten berechnet, ebenso wie Graphiken über die Verteilung der Häufigkeit und das zeitliche Zusammentreffen von Veränderungen bei den aufgezeichneten Signalen.

Das Diagnosesystem gemäß der vorliegenden Erfindung beinhaltet eine kostensparende, hoch-automatisierte, ambulante Diagnoseauswertung, die sich bei der Erkennung von Risikopatienten bewährt hat.

Die vorliegende Erfindung erlaubt ebenso eine exakte Unterscheidung zwischen jenen Patienten, welche Symptome aufweisen, die denen der Apnoe gleichen, jedoch eine von Apnoepatienten unterschiedliche Etiologie haben. Eine Therapie, die für diese Apnoepatienten angezeigt ist, kann daher schneller erkannt werden. Da die Erfindung darüberhinaus eine hochzuverlässige Methode zur Diagnose von Schlafapnoe darstellt, kann eine kostenaufwendige polysomnographische Auswertung auf solche Apnoeatienten beschränkt werden, die an organischer Apnoe leiden, für die weitere Tests zur Bestimmung der geeigneten Therapie notwendig sind.

Dadurch, daß durch diese ambulante Methode eine zuverlässige Diagnose aufgestellt werden kann und die Kosten und Unannehmlichkeiten einer stationären polysomnographischen Auswertung vermieden werden, kann durch die vorliegende Erfindung bei vielen Patienten die Schlafapnoe in einem früheren Stadium, nämlich schon dann, wenn die Behandlung einen höheren Grad an Effektivität verspricht, behandelt werden. Bei vielen Patienten wird auch die Gefahr von Komplikationen vermindert, da durch die kostengünstige, effektive Diagnose das Problem schon behandelt werden kann, bevor der Patient klinische Symptome entwickelt, die eine Uberweisung in ein Schlaflabor erfordern.

Ein weiterer Vorteil der vorliegenden Erfindung ist die Einfachheit des ambulanten Gerätes für diese hoch effektive, automatisierte Auswertung. Die Aufzeichnungseinheit gleicht in ihrer Form anderen Tonaufnahmegeräten und ihr Betrieb erfordert keinen Eingriff von seiten des Patienten. Der Aufzeichnungszeitraum wird vom medizinischen Personal im voraus eingestellt.

Ein weiterer Vorteil der vorliegenden Erfindung besteht darin, daß Datenübertragung, Datenauswertung, und die Erstellung von Graphiken und Berichten voll automatisiert sind. Alle erforderlichen Maßnahmen erfolgen als Reaktionen auf Bildschirmaufforderungen eines konventionellen Personal Computers. Im Gegensatz zu gängigen Diagnoseverfahren werden darüberhinaus nur selektierte, spezifische Informationen aufgezeichnet und weitergeleitet.

Es ist ein weiterer Vorteil der Erfindung, daß die angewendeten Überwachungssensoren äußerlich getragen werden und in ihrer Erscheinung unaufdringlich sind. Erwachsene Patienten können diese Sensoren ohne die Hilfe dritter anbringen und tragen. Falls erforderlich, kann der Patient die Sensoren und die Aufzeichnungseinheit auch unter normaler Straßenkleidung tragen.

### Kurze Beschreibung der Zeichnungen.

Eine Betrachtung der Beschreibung der bevorzugten Ausführung zusammen mit den Zeichnungen trägt zum besseren Verständnis der Merkmale und Vorteile der vorliegenden Erfindung bei.
- Abb. 1: ist eine perspektivische Darstellung des Überwachungsgerätes gemäß der vorliegenden Erfindung;
- Abb. 2: ist eine Prinzipskizze des Diagnosegerätes gemäß der vorliegenden Erfindung;
- Abb. 3: ist ein beispielhafter Diagnosebericht, der gemäß der bevorzugten Ausführung der vorliegenden Erfindung formatiert wurde;

### Beschreibung einer bevorzugten Ausführung.

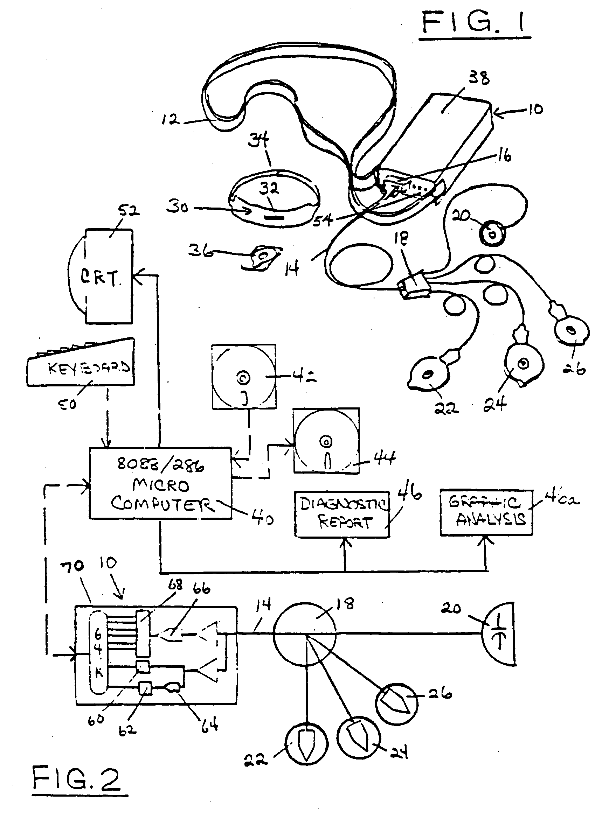

Das in Abb. 1 gezeigte Überwachungsgerät umfaßt eine leichte, batteriebetriebene Datenaufzeichnungseinheit 10 mit einem 64K RAM Speicher. Die Aufzeichnungseinheit ist mit einem Traggurt 12 versehen, mit dem der Patient die Einheit um die Schulter tragen kann. Die Aufzeichnungseinheit 10 besitzt kleine Ausmaße und hat ein sehr geringes Gewicht - eine Dicke von 30 mm und 160 x 90 mm auf der Oberseite und ihre Gewicht beträgt ohne Batterien etwa 300 g.

Ein Datenkabel 14 ist vorgesehen, welches einen mehrpoligen Stecker 16 hat, der mit Hilfe von zwei Schrauben an der Aufzeichnungseinheit 10 befestigt werden kann. Das Datenkabel 14 endet in einem Kabelverbindungskasten 18. Ein kleines, scheibenförmiges Elektretmikrophon 20 und drei EKG-Elektroden 22, 24, 26 sind an den Kabelverbindungskasten 18 angeschlossen.

Die Überwachungseinheit umfaßt weiterhin ein Polypropylenband 30 mit einem Schlitz 32, durch den das Elektretmikrophon 20 eingefügt wird, nachdem das Band mit Hilfe einer Klettverbindung 34 um den Hals des Patienten befestigt wurde. Das Band wird so eingestellt, daß das Mikrophon 20 am Kehlkopf des Patienten anliegt. Auf jener Seite des Mikrophones 20, welche am Kehlkpof des Patienten anliegt, befindet sich eine ringförmige, isolierende Polsterschicht, die von einem ringförmigen, selbsthaftenden Einwegschutz 36 umgeben ist. Der Traggurt 12 der Datenaufzeichnungseinheit 10 ist mit einem Schulterträger 38 verbunden, der ebenfalls isoliert und gepolstert ist, um jegliche Beschwerden während des Tragens auf ein Minimum zu reduzieren.

Das Signal der Atmungslaute vom Elektretmikrophon wird an zwei Schwellenwertdetektoren 60, 62 weitergeleitet. Durch den ersten Detektor 60 wird festgestellt, ob das akustische Signal vom Elektretmikrophone eine Stärke erreicht hat, die dem normalen Atmungslaut des Patienten entspricht. Dieses Signal des Elektretmikrophones wird ebenfalls einem zweiten Schwellenwertdetektor 62 zugeführt, durch einen Filter 64, der über 800 Hz 12 dB pro Oktavsignaldämpfung liefert. Der zweite Schwellenwertdetektor stellt fest, wenn dieses gefilterte Signal eine Stärke erreicht, die den Schnarchlauten des Patienten entspricht.

Das EKG-Spannungssignal wird praxisüblich über zwei aktive Elektroden 22 und 24, relativ zur Spannung einer Referenzelektrode 26, differenziert erfasst. Das Signal wird dann einem Spitzenwertdetektor zugeführt, der die Zeitabstände der Spitzenwerte zwischen den R-Wellen Spannungen im EKG mißt. Der Wert des letzten Intervalls wird jede Sekunde neu definiert und von einer Abtast-Halteschaltung 68 codiert.

Jeder diese Werte wird dann durch den RAM Speicher 70 als acht-bit Wort aufgezeichnet. Zwei Bits zeigen jeweils das Auftreten von Atmungslauten und Schnarchen während einer Abtastperiode an, und sechs Bits stellen den Wert des Spitzenwertabstands innerhalb einer Abtastperiode dar. Die Genauigkeit des so in der bevorzugten Ausführung der Erfindung codierten Herzfrequenzwertes ist in Tabelle 1 detailliert angegeben.

**Tabelle 1**

| Herzfrequenz | Ansprechempfindlichkeit |
|---|---|
| 0 - 20 bpm | 20 bpm |
| 21 - 30 bpm | 5 bpm |
| 31 - 130 bpm | 2 bpm |
| 131 - 160 bpm | 5 bpm |
| 161 - 200 bpm | 10 bpm |

Die Abtastwerte werden 18 Stunden lang von der Aufzeichnungseinheit 10 permanent gespeichert. Je nach den individuellen Schlafgewohnheiten des Patienten, wird der Beginn der Aufzeichnungsperiode im allgemeinen auf eine bestimmte Zeit zwischen 18.00 Uhr und Mitternacht des Tages festgelegt, an dem die Vorprogrammierung vorgenommen wurde.

Gemäß der bevorzugten Ausführung der vorliegenden Erfindung ist der Mehrpolstecker 16 der erwähnten Aufzeichnungseinheit 10 mit einem RS232 Standard, 9600 Baud Computer Interface und einem nicht näher dargestellten Schnittstellenkabel kompatibel. Nach Abschluß der Aufzeichnungen wird die Aufzeichnungseinheit 10 über diesen Stecker 16, wie in Abb. 2 dargestellt, an einen- vorzugsweise handelsüblichen IBM-XT oder AT kompatiblen - Personal Computer 40 angeschlossen. Aus Sicherheitsgründen wird derselbe Stecker 16 der Aufzeichnungseinheit 10 für beide Kabel verwendet, und der Patient kann daher nicht über die Aufzeichnungseinheit 10 an den Computer 40 angeschlossen werden, was möglicherweise zu einem gefährlichen elektrischen Schlag führen könnte.

Eine menü-geführte Datenanalyse und eine Formatierung des Berichts durch ein Programm gemäß der bevorzugten Ausführung der Erfindung sind auf einer Programmdiskette 42 verfügbar und können in den Mikrocomputer 40 geladen werden kann. Das Programm steuert automatisch alle Vorgänge, welche zu einer vorläufigen Bestimmung notwendig sind, die durch die Aufzeichnungseinheit 10 vorliegenden Daten werden analysiert, die Diagnose-Indizes und die statistischen Angaben für den Diagnosebericht 46 und die Graphik 46a werden errechnet und auf Befehl ausgedruckt.

Gemäß der bevorzugten Ausführung der Erfindung wird der Patient mit Verdacht auf Apnoe einer Voruntersuchung unterzogen, wobei Größe, Gewicht und Blutdruck des Patienten festgestellt werden. Die persönlichen Angaben des Patienten werden zusammen mit den Angaben über Größe, Gewicht und diastolischen Blutdruck vom medizinischen Personal auf Bildschirmaufforderungen hin über die Tastatur 50 in den Computer eingegeben.

Der Computer durchläuft dann ein schrittweises Interviewprogramm, das entweder auf einem gängigen Fragebogen zur Apnoevoruntersuchung basiert, welcher von der Deutschen Gesellschaft für Pneumologie entworfen wurde oder dem ein entsprechender Fragebogen über Anamnese zugrunde liegt.

Der Patient beantwortet die Fragen auf dem Bildschirm 52 entweder mit JA oder NEIN indem er auf der Tastatur 50 die Taste 1 oder 2 drückt. Beispiel:
1. Hat Ihr Partner bei Ihnen Atemstockungen während Ihres Schlafes bemerkt?
   ( ) 1 - JA (positive Indikation)
   ( ) 2 - NEIN (weiter)
2. Haben Sie Einschlafschwierigkeiten?
   ( ) 1 - JA ( -1 Punkt)
   ( ) 2 - NEIN ( 0 Punkte)
3. Nehmen Sie Schlafmittel?
   ( ) 1 - JA ( -1 Punkt)
   ( ) 2 - NEIN ( 0 Punkte)
4. Zweimal JA/einmal JA ( +1 Punkt)
   Zweimal NEIN ( 0 Punkte)
   a. Schnarchen Sie oft?
      ( ) 1 - JA
      ( ) 2 - NEIN
   b. Wenn Sie schnarchen, schnarchen Sie laut und unregelmäßig?
      ( ) 1 - JA
      ( ) 2 - NEIN
5. Ein bis dreimal JA ( +1 Punkt)
   Dreimal NEIN ( 0 Punkte)
   a. Schlafen Sie tagsüber leicht ein?
      ( ) 1 - JA
      ( ) 2 - NEIN
   b. Fällt es Ihnen schwer wach zu bleiben, selbst wenn Sie nicht völlig entspannen wollen, z.b. beim Lesen oder Fernsehen?
      ( ) 1 - JA
      ( ) 2 - NEIN
   c. Fühlen Sie sich oft müde und erschöpft?
      ( ) 1 - JA
      ( ) 2 - NEIN

Die Antworten des Patienten und sein physischer Zustand werden dann durch den Computer, wie in Tabelle 2 dargestellt, automatisch zusammengezählt.

**Tabelle 2**

| Erreichte Punktzahl | Aussage |
|---|---|
| -3, -2 oder-1 | Indikation negativ, Befund unwahrscheinlich |
| 0 oder +1 | Indikation positiv, Befund unsicher |
| +2 oder+3 | Indikation positiv, Befund wahrscheinlich. |

Der Computer zeigt an, ob der Apnoebefund, der automatisch aus der Punktezahl des Patienten, verglichen mit einer im Programm beinhalteten Tabelle, resultiert, "wahrscheinlich" oder "unsicher" oder "unwahrscheinlich" ist.

Gemäß der bevorzugten Ausführung werden Aufzeichnungseinheiten nur dann eingesetzt, wenn der Computer einen Verdacht auf Apnoe als "wahrscheinlich" oder "unsicher" anzeigt, wodurch weitere Tests vermieden werden, wenn eine Apnoe unwahrscheinlich ist. Vor Beginn der Aufzeichnungsperiode wird die Aufzeichnungseinheit dann vorprogrammiert, der Patient wird im Gebrauch der Sensoren unterrichtet und ein Folgetermin zur Datenauswertung wird vereinbart.

Die Aufzeichnungseinheit wird automatisch vorprogrammiert, in dem sie über das nicht dargestellte Schnittstellenkabel und den Computer 40 angeschlossen wird. Als Antwort auf Bildschirmaufforderungen wählt das medizinische Personal die Zeit für den Aufzeichnungsbeginn und gibt das Datum und die Kennnummer der Aufzeichnungsperiode per Tastatur 50 ein. Der Computer löscht alle vorhergehenden Daten, die noch in der Aufzeichnungseinheit 10 enthalten sind und speichert einen 256 Zeichen umfassenden Kennsatz im Speicher der Aufzeichnungseinheit 10. Das Programm entnimmt den Kennsatz automatisch den eingegebenen Daten der Voruntersuchung und gewährleistet so eine korrekte Identifizierung des Aufzeichnungszeitraumes und des Patienten.

Der Patient wird dann unterrichtet, wie Mikrophon und EKG-Elektroden während der Vorprogrammierung anzubringen sind. Nach dem Anbringen von Mikrophon und Elektroden wird die Funktionsfähigkeit der Einheit mit Hilfe von drei LED Anzeigen 54, welche in der Aufzeichnungseinheit 10 untergebracht sind, getestet. Eine der Anzeigen spricht an, wenn der Patient Schnarchlaute simuliert, eine andere leuchtet auf bei normalen Atmungslauten, bleibt jedoch dunkel bei zeitweisem Atmungsstillstand. Getestet werden auch Mikrophone, Straßenverstärker, Filterung und Detektorschaltungen. Die dritte Anzeige spricht im gleichen Rhythmus wie der Herzschlag des Patienten an, der durch die Spitzenwerte der Signale der EKG-elektroden wiedergegeben wird. Durch eine Überprüfung dieses Rhythmus dieser Anzeige werden die Sensorelektroden und Detektorschaltungen für die Spitzenwerte des EKG-Verstärkers getestet.

Jedesmal wenn das Datenkabel 14 an die Aufzeichnungseinheit 10 angeschlossen wird, sind diese drei Leuchten 54 für die Dauer von 5 Minuten aktiv. Nach 5 Minuten schalten sich die Leuchten automatisch ab, um Energie für die Batterien zu sparen.

Beim Folgetermin ruft das medizinische Personal die Daten der Voruntersuchung des Patienten aus dem Speicher ab und formatiert eine neue Patientendiskette 44 gemäß den Programmaufforderungen auf dem Bildschirm. Das Programm zeichnet einen 256-Zeichen Kennsatz automatisch auf der Diskette auf. Dieser Kennsatz wird von den Voruntersuchungsdaten des Patienten auf die gleiche Art abgeleitet, wie jener Kennsatz, der durch den Computer in der Aufzeichnungseinheit während der Vorprogrammierung dem Speicher 70 der Aufzeichnungseinheit 10 vor Beginn der Aufzeichnungsperiode zugeführt wird.

Wenn die Aufzeichnungseinheit 10 an den Computer 40 angeschlossen wird, testet das Analyseprogramm auf der Programmdiskette 42 zuerst, ob der soeben auf der Patientendiskette 44 aufgezeichnete Kennsatz mit dem alten Kennsatz im Speicher der Aufzeichnungseinheit 10 übereinstimmt. Stimmen die Kennsätze überein, so kopiert das Programm die Rohdaten der Aufzeichnungseinheit 10 über das Schnittstellenkabel (nicht dargestellt) auf die vorformatierte 360kB Patientendiskette 44. Dieser Vorgang dauert etwa 90 Sekunden.

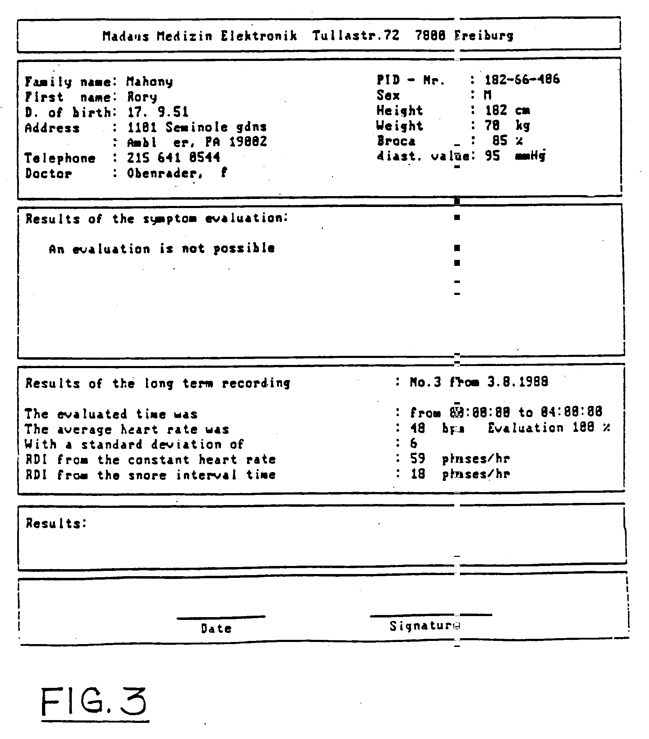

Nach dem Kopieren der aufgezeichneten Signale, berechnen die Programme zwei Atmungsstörungsindizes (RDI) von den aufgezeichneten Daten, um einen Diagnosebericht auszudrucken, so wie der beispielhafte Bericht in Abb. 3, und um aus den Rohdaten Graphiken zu formatieren und die Daten statistisch zu analysieren.

Der erste RDI-Wert gibt die Anzahl der Episoden/Stunden ohne Atmungslaute an. Der zweite RDI-Wert stellt die Anzahl der Episoden pro Stunde dar, in denen die Pulsfrequenz des Patienten zwischen 90 % und 109% ihres Durchschnittes lag. Diese Durchschnitts-frequenz wird für jede Aufzeichnungsperiode berechnet. Das Zeitkriterium für beide RDI-Werte beschränkt die Indizierung auf jene Episoden, deren Dauer zwischen 11 und 60 Sekunden liegt. Episoden, die kürzer oder länger dauern, werden nicht in die Bewertung miteinbezogen. Diese Werte werden unter dem Abschnitt "Ergebnisse der Langzeit-Aufzeichnung" in Abb. 3 aufgeführt.

Unter dem Abschnitt "Ergebnisse der Symptom-Bewertung" hält das Programm die Ergebnisse aus dem Fragebogen fest und und gibt Punkte von "0" or "±1" in der Kategorie in Tabelle 3.

**Tabelle 3**

| | |
|---|---|
| 1. | Einschlafschwierigkeiten |
| 2. | Schlafmittel |
| 3. | Schnarchen |
| 4. | Vigilanz |
| 5. | Broca Index, diastolische Werte (Fitness) |

Der "Broca Index" ist ein Index, der sich aus dem Verhältnis zwischen Gewicht und Größe des Patienten ergibt. Der andere Fitness-Faktor ist der diastolische Blutdruck. Wird der Voruntersuchungsfragebogen außer Acht gelassen, druckt der Computer "eine Bewertung ist nicht möglich" anstelle einer Bewertung dieses Symptoms.

## Patentansprüche

1. Ambulantes Diagnosesystem zur unverzüglichen Behandlung von Schlafapnoe mit
Mitteln (22, 24, 26) zur Abtastung der Herzspannung;
Mitteln (66) zur Bestimmung der Herzfrequenz aus der Herzspannung;
Mitteln (70) zur Aufzeichnung kodierter Signale, welche die Herzfrequenz darstellen, dadurch gekennzeichnet, daß es umfaßt:
Mittel (20) zur Erfassung von Atmungslauten;
Mittel (20) zur Erfassung von Schnarchlauten;
Mittel (70) zur Aufzeichnung kodierter Signale, welche die Atmungs- und Schnarchlaute darstellen;
Mittel (40) zur Berechnung des Atmungsregulationsstörungs-indexes aus der Anzahl der Abschnitte, in denen die Herzfrequenz im wesentlichen durchschnittlich war und der Anzahl der Abschnitte, in denen Atmungslaute nicht erfaßt wurden;
Mittel (40) zur Formatierung der aufgezeichneten Signale zur Erstellung von Graphiken über die zeitliche Beziehung zwischen den Veränderungen, die in der aufgezeichneten Herzfrequenz des Patienten von Schnarch- und Atmungslauten des Patienten auftreten; und
Mittel (40) zur Formatierung statistischer Graphiken aus den aufgezeichneten Signalen, wobei diese Graphiken die Herzfrequenzverteilung ebenso wiedergeben wie die Häufigkeitsverteilung der Dauer der Zeitabschnitte zwischen dem aufgezeichneten Auftreten von Schnarchlauten und der Dauer der Zeitabschnitte, in denen die Herzfrequenz konstant bleibt.

2. Ambulantes Diagnosesystem gemäß Anspruch 1, dadurch gekennzeichnet, daß die Mittel (22, 24, 26) zur Abtastung der Herzspannung drei EKG-Elektroden, welche äußerlich getragen werden, umfassen.

3. Ambulantes Diagnosesystem gemäß Anspruch 1 oder 2, dadurch gekennzeichnet, daß die Mittel (20) zur Erfassung von Atmungslauten ein Elektretmikrophon umfassen, das so angepaßt wurde, daß der Patient es am Kehlkopf tragen kann.

4. Ambulantes Diagnosesystem gemäß einem der vorhergehenden Ansprüche, dadurch gekennzeichnet, daß die Mittel (70) zur Aufzeichnung der kodierten Signale so angepaßt wurden, daß der Patient sie in einem Träger unter der Straßenkleidung tragen kann.

5. System gemäß einem der vorhergehenden Ansprüche, dadurch gekennzeichnet, daß die Zeitabschnitte, die zur Berechnung des Indexes der Atmungsregulationsstörung ermittelt werden, länger als 10 Sekunden, jedoch nicht länger als 1 Minute dauern.

## Claims

1. An ambulant diagnostic system for the immediate treatment of sleep apnea, comprising
- means (22, 24, 26) for scanning the voltage of the heart;
- means (66) for determining the heart rate from the heart voltage;
- means (70) for recording coded signals representing the heart rate,
characterised in that it further comprises:
- means (20) for detecting sounds of breathing;
- means (20) for detecting sounds of snoring;
- means (70) for recording coded signals representing sound of breathing and snoring;
- means (40) for calculating the disturbed respiration regulation index from the number of periods in which the heart rate was substantially average and the number of periods in which no sound of breathing was detected;
- means (40) for formatting the recorded signals for establishing graphs on the time relationship of the changes occurring in the recorded heart rate of the patient during sounds of snoring and breathing of the patient; and
- means (40) for formatting statistic graphs from the recorded signals, these graphs representing the heart rate distribution, as well as the frequency distribution of the duration of the periods between the recorded occurrences of snoring and the duration of periods in which the heart rate remains constant.

2. The ambulant diagnostic system of claim 1, characterised in that said means (22, 24, 26) for scanning the heart voltage comprise three ECG electrodes carried on the outside.

3. The ambulant diagnostic system of claim 1 or 2, characterised in that said means (20) for detecting sounds of breathing comprise an electret microphone adapted such that the patient can carry it on the larynx.

4. The ambulant diagnostic system of one of the preceding claims, characterised in that said means (70) for recording the coded signals are adapted such that the patient may carry it in a carrier under the street wear.

5. The ambulant diagnostic system of one of the preceding claims, characterised in that the time intervals determined to calculate the respiration regulation disturbance index lasts longer than 10 seconds, yet not longer than 1 minute.

## Revendications

1. Système de diagnostic ambulatoire pour le traitement instantané de l'apnée du sommeil comprenant
des moyens (22, 24, 26) pour l'exploration du potentiel cardiaque,
des moyens (66) pour la détermination de la fréquence cardiaque à partir du potentiel cardiaque ;
des moyens (70) pour l'enregistrement de signaux codés représentant la fréquence cardiaque, caractérisé en ce qu'il comprend
un moyen (20) pour la détection des bruits de respiration ;
un moyen (20) pur la détection des bruits de ronflement ;
un moyen (70) pur l'enregistrement de signaux codés représentant les bruits de respiration et de ronflement ;
un moyen (40) pour le calcul de l'indice de perturbation de la régulation de la respiration à partir du nombre des intervalles de temps pendant lesquels la fréquence cardiaque était sensiblement moyenne et du nombre d'intervalles de temps pendant lesquels les bruits de respiration n'ont pas été détectés ;
un moyen (40) pour la mise en forme des signaux enregistrés pour l'établissement de graphiques concernant la relation en fonction du temps entre les variations qui, dans la fréquence cardiaque enregistrée du patient, proviennent des bruits de ronflement et de respiration du patient, et
un moyen (40) pour la mise en forme de graphiques statistiques à partir des signaux enregistrés, ces graphiques reproduisant la distribution des fréquences cardiaques ainsi que la distribution de fréquences de la durée des périodes entre l'apparition des bruits de ronflement enregistrée et de la durée des périodes pendant lesquelles la fréquence cardiaque demeure constante.

2. Système de diagnostic ambulatoire selon la revendication 1, caractérisé en ce que les moyens (22, 24, 26) pour l'exploration du potentiel cardiaque comportent trois électrodes d'ECG portées extérieurement.

3. Système de diagnostic ambulatoire selon la revendication 1 ou 2, caractérisé en ce que les moyens (20) pour la détection des bruits de respiration comprennent un microphone à électret qui a été adapté de telle manière que le patient puisse le porter sur le larynx.

4. Système de diagnostic ambulatoire selon l'une des revendications précédentes, caractérisé en ce que les moyens (70) pour l'enregistrement des signaux codés ont été adaptés de telle manière que le patient puisse les porter dans une bride sous le costume de ville.

5. Système selon l'une des revendications précédentes, caractérisé en ce que les intervalles de temps qui sont déterminés pour le calcul de l'indice de perturbation de la régulation de la respiration durent plus de 10 secondes, mais pas plus de 1 minute.
